(19) Europäisches Patentamt / European Patent Office / Office européen des brevets

(11) **EP 3 968 860 B1**

(12) **FASCICULE DE BREVET EUROPEEN**

(45) Date de publication et mention
de la délivrance du brevet:
**09.10.2024 Bulletin 2024/41**

(21) Numéro de dépôt: **20737238.4**

(22) Date de dépôt: **14.05.2020**

(51) Classification Internationale des Brevets (IPC):
**A61B 8/00** *(2006.01)*     **A61B 8/08** *(2006.01)*
**G06T 7/20** *(2017.01)*

(52) Classification Coopérative des Brevets (CPC):
**A61B 8/485; A61B 8/4245; A61B 8/5276;**
**G06T 5/50; G06T 5/73; G06T 7/269;**
G06T 2207/10016; G06T 2207/10132;
G06T 2207/20182; G06T 2207/20201

(86) Numéro de dépôt international:
**PCT/FR2020/050807**

(87) Numéro de publication internationale:
**WO 2020/234530 (26.11.2020 Gazette 2020/48)**

(54) **PROCÉDÉ POUR DÉTERMINER PAR ULTRASONS UNE IMAGE CORRIGÉE D'UN MILIEU, ET DISPOSITIF POUR METTRE EN OEUVRE CE PROCÉDÉ**

VERFAHREN ZUR ULTRASCHALLBESTIMMUNG EINES KORRIGIERTEN BILDES EINES MEDIUMS UND VORRICHTUNG ZUR DURCHFÜHRUNG DIESES VERFAHRENS

METHOD FOR ULTRASOUND DETERMINATION OF A CORRECTED IMAGE OF A MEDIUM, AND DEVICE FOR IMPLEMENTING THIS METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.05.2019 FR 1905212**

(43) Date de publication de la demande:
**23.03.2022 Bulletin 2022/12**

(73) Titulaire: **SUPERSONIC IMAGINE**
**13290 Aix en Provence (FR)**

(72) Inventeur: **COUADE, Mathieu**
**13090 Aix en Provence (FR)**

(74) Mandataire: **Plasseraud IP**
**104 Rue de Richelieu**
**CS92104**
**75080 Paris Cedex 02 (FR)**

(56) Documents cités:
**EP-A1- 2 535 004       US-A1- 2011 028 838**
**US-A1- 2011 301 465       US-A1- 2015 146 953**

• **O'DONNELL M ET AL: "Internal displacement and strain imaging using ultrasonic speckle tracking", IEEE TRANSACTIONS ON ULTRASONICS, FERROELECTRICS AND FREQUENCY CONTROL, IEEE, US, vol. 41, no. 3, 1 May 1994 (1994-05-01), pages 314 - 325, XP011439071, ISSN: 0885-3010, DOI: 10.1109/58.285465**

**Description**

**DOMAINE TECHNIQUE**

La présente divulgation est relative aux procédés et dispositifs d'imagerie par ondes ultrasonores pour fournir des images d'un milieu viscoélastique. Notamment, le procédé a pour but de corriger les images produites en prenant en compte une variation de pression exercée par une sonde ultrasonore d'imagerie sur une surface externe du milieu.

**ETAT DE LA TECHNIQUE ANTERIEURE**

Plus particulièrement, la présente divulgation concerne un procédé d'imagerie utilisant des ondes ultrasonores pour observer un milieu viscoélastique diffusant qui contient des particules réfléchissantes desdites ondes ultrasonores de compression.

Les images corrigées par le procédé peuvent être de divers type. Par exemple, les images peuvent être de type déformation (« strain » en langue anglaise) ou de type élasticité (« elasticity » en langue anglaise).

Le document US 2005/252295 propose une technique d'imagerie d'élasticité pour laquelle on génère une onde de cisaillement élastique dans le milieu, et on observe la propagation de cette onde de cisaillement élastique en enregistrant une pluralité d'images du milieu. On détermine alors des images d'au moins un paramètre de mouvement du milieu (déplacement, déformation partielle), et on en déduit des images d'un paramètre de propagation de l'onde de cisaillement, tel que la vitesse de l'onde de cisaillement, le module de cisaillement, le module d'Young, l'élasticité de cisaillement, etc...

Cette dernière technique est très utilisée. Cependant, il a été constaté que les images du paramètre de mouvement ou les images du paramètre de propagation de l'onde de cisaillement sont sensibles à la pression exercée par la sonde ultrasonore d'imagerie sur la surface du milieu. Or, cette pression de la sonde est exercée par le praticien et peut varier lors d'un examen ou d'un examen à un autre. En effet, le praticien fait varier cette pression externe pour obtenir l'observation désirée sur l'image considérée.

En outre, ces images comprennent des déformations liées aux variations de la pression externe exercée, ce qui génère des imprécisions dans leur représentation, dans leur reproductibilité et dans leur détermination.

Le document US 2015/146953 divulgue un procédé (et le dispositif correspondant) pour déterminer par ultrasons une image corrigée d'un milieu, dans lequel une succession temporelle d'images du milieu est utilisée pour déterminer une image de déformation, ladite image étant corrigée prenant en compte le mouvement la sonde pendant l'acquisition d'images.

**EXPOSE DE L'INVENTION**

La présente invention a pour but de perfectionner les procédés d'imagerie de ce type, notamment pour en améliorer les images.

A cet effet, la présente divulgation propose un procédé pour déterminer par ultrasons une image corrigée définit dans la revendication 1.

La présente divulgation se rapporte également à un dispositif d'imagerie définit dans la revendication 15.

**BREVE DESCRIPTION DES DESSINS**

D'autres caractéristiques et avantages de la présente divulgation apparaîtront au cours de la description suivante de divers modes de réalisation, donnés à titre d'exemple non limitatif, en regard des dessins joints.

Sur les dessins :

- la figure 1 est une vue schématique d'un dispositif d'imagerie par ultrasons selon une forme de réalisation de la divulgation ;
- les figures 2A et 2B montrent de manière schématique le mouvement d'une image du milieu entre une pression externe nulle et une pression externe qui déforme la surface externe du milieu ;
- la figure 2C montre de manière schématique un mouvement plus complexe que celui de la figure 2B, ce mouvement incluant une rotation ;
- la figure 3 est un diagramme présentant une forme de réalisation du procédé de calcul d'une image de déformation corrigée ;
- la figure 4 est un diagramme présentant un procédé pour déterminer une image d'élasticité corrigée ;
- la figure 5 est un diagramme présentant une première forme de réalisation d'un procédé pour déterminer une image d'un paramètre de non linéarité corrigé ;

- la figure 6 est un graphique présentant un exemple de mise en oeuvre du procédé de la figure 5 ;
- la figure 7A est un diagramme présentant un procédé pour déterminer une image de contrainte corrigée ;
- la figure 7B est un diagramme présentant une deuxième forme de réalisation d'un procédé pour déterminer une image d'un paramètre de non linéarité corrigé ; et
- la figure 8 est un graphique présentant un exemple de mise en oeuvre du procédé de la figure 7B.

Sur les différentes figures, fournies à titre d'illustration, les mêmes références numériques désignent des éléments identiques ou similaires.

## DESCRIPTION DETAILLEE

### Dispositif d'imagerie 1

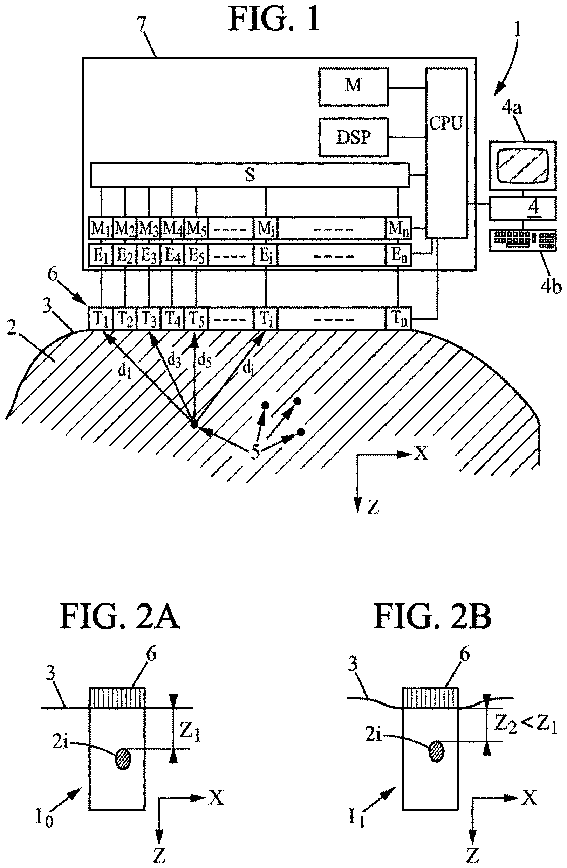

Le dispositif d'imagerie 1 représenté sur la **figure 1** est destiné à fournir des images d'un milieu viscoélastique 2 qui est diffusant vis à vis des ondes ultrasonores de compression, et qui peut être par exemple un corps vivant, par exemple une partie du corps d'un patient (sein, foie, abdomen, ...), dans le cas des applications médicales. Ce dispositif d'imagerie 1 est également apte à étudier la propagation des ondes élastiques de cisaillement pour fournir des images d'élasticité du milieu 2.

Les images du milieu sont produites par exemple au moyen d'un micro-ordinateur 4 (comprenant au moins une interface d'entrée 4b telle qu'un clavier ou autre, et une interface de sortie 4a telle qu'un écran ou autre) ou toute autre unité centrale électronique, qui fait envoyer dans le milieu 2, à partir de sa surface extérieure 3, des ondes ultrasonores de compression qui interagissent avec les particules diffusantes 5 contenues dans le milieu 2, lesquelles particules sont réfléchissantes pour les ondes ultrasonores de compression. Les particules 5 peuvent être constituées par toute hétérogénéité du milieu 2, et notamment, lorsqu'il s'agit d'une application médicale, par des particules de collagène présentes dans les tissus humains (ces particules forment sur les images échographiques des points connus sous le terme "speckle").

Pour observer le milieu 2 et pour générer des images du milieu, on utilise une sonde ultrasonore 6 disposée contre la surface extérieure 3 du milieu observé 2. Cette sonde envoie, selon un axe Z, des impulsions d'ondes ultrasonores de compression du type de celles couramment utilisées en échographie, à une fréquence comprise par exemple entre 0,5 et 100 MHz et de préférence entre 0,5 et 15 MHz, par exemple de l'ordre de 4 MHz.

La sonde ultrasonore 6 est constituée par un réseau de n transducteurs ultrasonores T1, T2, ..., Ti, ..., Tn, n étant un nombre entier supérieur ou au moins égal à 1.

Cette sonde 6 peut se présenter par exemple sous la forme d'une barrette linéaire pouvant comprendre par exemple n = 128 transducteurs alignés selon un axe X perpendiculaire à l'axe Z. La sonde en question peut être également un réseau bidimensionnel (plan ou non) de transducteurs.

Les transducteurs T1, T2, ... Tn peuvent être commandés indépendamment les uns des autres par le micro-ordinateur 4, éventuellement par l'intermédiaire d'une unité centrale CPU qui est contenue par exemple dans une baie électronique 7 reliée par un câble souple à la sonde 6. Les transducteurs T1-Tn peuvent ainsi émettre sélectivement :

- soit une onde ultrasonore de compression "plane" (c'est à dire en l'occurrence une onde dont le front d'onde est rectiligne dans le plan X, Z) ou tout autre type d'onde non focalisée éclairant l'ensemble du champ d'observation dans le milieu 2, par exemple une onde générée en faisant émettre des signaux acoustiques aléatoires par les différents transducteurs T1-Tn,
- soit une onde ultrasonore de compression focalisée en un ou plusieurs points du milieu 2.

Le document US 2009/234230 propose une technique d'imagerie synthétique utilisant plusieurs ondes de compression non focalisées, par exemple des ondes de type onde planes d'angles différents, et qui combine les ondes en retour de ces ondes planes pour obtenir de manière très rapide une image du milieu de qualité améliorée.

### Détermination d'images du milieu $I_k$

Pour générer une image du milieu (I) le dispositif d'imagerie effectue par exemple les étapes suivantes :

(i1) le micro-ordinateur 4 fait émettre dans le milieu viscoélastique, par la sonde 6 au moins un tir d'onde ultrasonore de compression non focalisée,
(i2) le micro-ordinateur 4 fait détecter par la sonde 6 et enregistrer en temps réel des signaux acoustiques reçus du milieu viscoélastique 2, comprenant les échos générés par l'onde ultrasonore de compression non focalisée en interagissant avec les particules réfléchissantes 5 du milieu viscoélastique, et

(i3) une étape de traitement au cours de laquelle le micro-ordinateur 4 traite les signaux acoustiques reçus du milieu viscoélastique 2 au cours de la sous-étape (i2) pour déterminer une ou plusieurs images du milieu (I).

L'onde ultrasonore de compression non focalisée se propage dans le milieu 2 avec une vitesse de propagation très élevée, par exemple de l'ordre de 1500 m/s dans le corps humain, et interagit avec les particules réfléchissantes 5, ce qui génère des échos ou autres perturbations analogues du signal, connus en soi sous le nom de "bruits de speckle" dans le domaine de l'échographie.

Ces "bruits de speckle" sont captés par les transducteurs T1, ... , Tn au cours de la sous-étape (i2), après le tir d'onde ultrasonore de compression non focalisée. Le signal si(t) ainsi capté par chaque transducteur Ti est tout d'abord échantillonné à haute fréquence (par exemple de 30 à 100 MHz) et numérisé en temps réel par un échantillonneur appartenant à la baie 7 et relié à ce transducteur, respectivement E1, E2, ... En.

Le signal si(t) ainsi échantillonné et numérisé est ensuite mémorisé, également en temps réel, dans une mémoire Mi appartenant à la baie 7 et propre au transducteur Ti.

Chaque mémoire Mi présente par exemple une capacité de l'ordre de 128 Mo, et contient l'ensemble des signaux si(t) reçus.

Après la mémorisation de tous les signaux si(t), l'unité centrale CPU fait retraiter ces signaux par un circuit sommateur S appartenant à la baie 7 (ou bien elle effectue elle-même ce traitement, ou encore ledit traitement peut être effectué dans le micro-ordinateur 4), par un processus classique de formation de voies correspondant à la sous-étape (i3).

Des signaux S(x, z) sont ainsi générés, ces signaux correspondant chacun à l'image du champ d'observation du milieu après le tir de l'onde ultrasonore non focalisée.

Par exemple, on peut déterminer un signal S(t) par la formule suivante :

$$S(x, z, t) = \sum_{i=1}^{n} \alpha_i(x, z) . si[t(x, z) + d_i(x, z)/V]$$

dans laquelle :

si est le signal brut perçu par le transducteur n° i après le tir d'onde ultrasonore de compression,

t(x,z) est le temps mis par l'onde ultrasonore de compression pour atteindre le point du champ d'observation de coordonnées (x,z), avec t = 0 au début du tir,

di(x,z) est la distance entre le point du champ d'observation de coordonnées (x,z) et le transducteur n° i, ou une approximation de cette distance,

V est la vitesse moyenne de propagation des ondes acoustiques ultrasonores de compression dans le milieu viscoélastique observé, et

$\alpha i(x,z)$ est un coefficient de pondération tenant compte de lois d'apodisation (en pratique, on pourra dans de nombreux cas considérer que $\alpha i(x,z)$ = 1).

La formule ci-dessus s'applique mutatis mutandis lorsque le champ d'observation est à 3 dimensions (réseau bidimensionnel de transducteurs), en remplaçant les coordonnées spatiales du plan (x,z) par des coordonnées spatiales (x,y,z).

Après l'étape optionnelle de formation de voies, l'unité centrale CPU mémorise dans une mémoire centrale M appartenant à la baie 7 les signaux d'images S(x,z) correspondent au dernier tir. Ces signaux peuvent également être mémorisés dans le micro-ordinateur 4 afin qu'il effectue lui-même le calcul de l'image du milieu (I).

D'autres techniques de génération d'image du milieu (I) existent, comme les techniques d'imagerie synthétique. Toutes technique d'imagerie permettant d'obtenir des images du milieu est utilisable. De préférence on utilisera une technique permettant d'obtenir des images à grande cadence.

Le dispositif d'imagerie 1 et le procédé selon la présente divulgation effectuent une succession temporelle d'images du milieu pour suivre la variation de la pression externe P exercée sur la surface externe 3. Ainsi, on considérera qu'un nombre N+1 d'images du milieu sont prises à des instants temporels successifs, ces instants temporels n'étant pas obligatoirement séparés d'une période constante. Les images du milieu peuvent donc être identifiées par un indice d'image k compris entre 0 et N.

Les images du milieu seront donc identifiées par la notation $I_k$.

Cet indice k sera utilisé pour toute autre image ou grandeur déterminée à partir de l'image du milieu d'indice k et éventuellement d'images du milieu précédente. Cet indice pourra donc être utilisé pour repérer l'instant temporel associé à cette image du milieu d'indice k.

En outre, la première image de cette succession d'images est notée par convention l'image du milieu d'indice 0. On supposera que pour cette première image, la pression externe P est faible ou nulle, et que la déformation induite de la surface externe 3 est faible ou nulle. Cette première image est par exemple représentée en figure 2A.

Les **figures 2A à 2C** illustrent le problème résolu par la présente divulgation.

En figure 2A est représentée une utilisation du dispositif d'imagerie 1, la sonde 6 exerçant une pression externe P faible ou nulle sur la surface externe 3. La surface externe 3 demeure sensiblement horizontale (dans la direction X). L'image du milieu $I_0$ comprend par exemple une inclusion 2i à une profondeur 21 par rapport à la surface externe 3.

En figure 2B est représentée une utilisation du dispositif d'imagerie 1 à un instant temporel ultérieur de celui de la figure 2A, et avec une pression externe P' supérieure à P, qui déforme la surface externe 3 dans la direction Z vers l'intérieur du milieu 2. Le dispositif d'imagerie 1 produit une image du milieu $I_1$ de même taille que l'image $I_0$ produite en figure 2A, mais les couches supérieures du milieu au-dessus de l'inclusion 2i sont naturellement plus comprimées que les couches inférieures du milieu 2 en dessous de l'inclusion 2i, de sorte que l'inclusion 2i est dans cette image du milieu $I_1$ de la figure 2B déplacée vers la sonde 6 en comparaison de la première image $I_0$ de la figure 2A. Autrement dit, l'inclusion 2i est alors à une profondeur Z2 par rapport à la surface externe 3 inférieure à la profondeur 21 de la première image $I_0$.

Par conséquent, pendant une variation de la pression externe, les éléments internes du milieu se déplacent dans l'image du milieu à cause de l'élasticité dudit milieu 2. Ainsi, il est nécessaire de connaître ces mouvements généraux pour effectuer un suivi et une analyse précise de caractéristiques de ces éléments internes.

L'exemple des figures 2A et 2B montre qu'une translation dans la direction Z pourrait donner une correction adéquate.

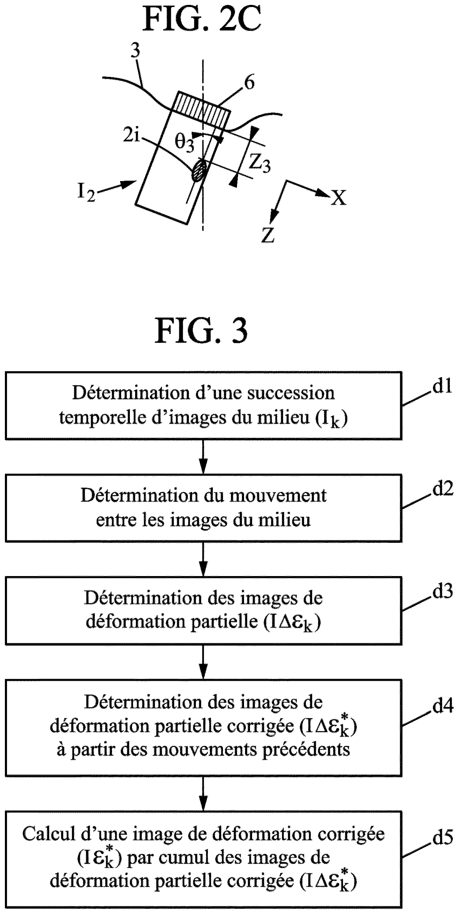

En figure 2C est représenté une utilisation du dispositif d'imagerie 1 à un autre instant temporel, par exemple encore ultérieur aux précédents instants temporels représentés aux figures 2A et 2B, et avec une pression externe P différente, et surtout un basculement de la sonde 6 par rapport à la direction verticale Z (basculement exagéré sur cette figure 2C). La surface externe 3 est déformée en direction Z et elle est inclinée d'un angle Θ. Le dispositif d'imagerie 1 produit une image du milieu $I_2$ de même taille, mais les couches supérieures du milieu sont aussi comprimées et l'inclusion 2i est déplacée latéralement dans l'image, c'est-à-dire déplacée en direction X par l'inclinaison de la sonde 6.

Ainsi, les variations de pression et de position de la sonde 6 sur la surface externe 3 déplacent les éléments internes du milieu dans l'image du milieu et il apparait nécessaire de connaître ces mouvements généraux (très lents par rapport aux ondes ultrasonores) pour en effectuer un suivi et/ou pour les compenser dans les images du milieu prises afin d'effectuer une analyse précise de caractéristiques des éléments internes.

La sonde 6 peut éventuellement avoir un déplacement plus complexe qu'une simple translation en direction Z ou qu'une combinaison d'une translation et d'une rotation. Le mouvement de correction nécessaire sera alors plus complexe. Les détails de la détermination de ces mouvements seront explicités plus loin dans la présente divulgation.

## Détermination d'un champ de déplacement $u_k$

Les images du milieu $(I_k)$ peuvent être traitées par corrélation et avantageusement par intercorrélation soit deux à deux, c'est-à-dire entre une image du milieu d'indice k $(I_k)$ et l'image du milieu du milieu d'indice k-1 $(I_{k-1})$.

L'intercorrélation peut être réalisée par exemple dans un circuit électronique spécialisé DSP appartenant à la baie 7, ou être programmée dans l'unité centrale CPU ou dans le micro-ordinateur 4.

Au cours de ce processus d'intercorrélation, on maximise une fonction d'intercorrélation **<$S_{k-1}(x,z)$ ,$S_k(x,z)$>** afin de déterminer le déplacement subi par chaque particule 5 donnant lieu à un écho ultrasonore.

Des exemples de tels calculs d'intercorrélation sont donnés dans l'état de la technique, notamment dans les documents suivants :

- "Internal displacement and strain imaging using speckle tracking", O'Donnell et al, IEEE transactions on ultrasonic, ferroelectrics, and frequency control, vol. 41, n° 3, mai 1994, p. 314-325 ),
- "Elastography: a quantitative method for imaging the elasticity of biological tissues", Ophir et al., Ultrasonic imaging., vol. 13, p.111-134, 1991), et
- "Pyramidal Implementation of the Lucas Panade Feature Tracter. Description of the algorithm.", J-Y Bouguet, Intel Corp.

On obtient ainsi un champ de déplacement, c'est à dire un ensemble de vecteurs déplacements ou u(x,z,t) en chaque position (x,z) du milieu 2, que l'on peut noter $u_k(x,z)$ en remplaçant la variable de l'instant temporel t par l'indice d'image k. Ces vecteurs déplacements peuvent éventuellement être réduits à une seule composante ou à deux ou trois composantes. Dans l'exemple considéré, le champ de déplacement $u_k$ à l'indice d'image k est :

$$u = \begin{bmatrix} u_x \\ u_z \end{bmatrix}$$

Ce champ de déplacement (ensemble de vecteurs déplacements) est stocké dans la mémoire M ou dans le micro-ordinateur 4.

On peut construire une image du champ de déplacement $Iu_k$, et l'ensemble des images du champ de déplacement $Iu_k$ correspondant à la succession temporelle des images du milieu peuvent être visualisés, notamment au moyen de l'écran 4a du micro-ordinateur, par exemple, sous la forme d'un film ralenti où la valeur des déplacements est illustrée par un paramètre optique tel que par un niveau de gris ou par un niveau chromatique.

## Détermination d'images de déformation partielle $I\Delta\varepsilon_k$

On peut alors calculer les déformations partielles $\Delta\varepsilon$ en chaque point du milieu 2, c'est à dire des vecteurs dont les composantes sont les dérivées des composantes des vecteurs déplacements respectivement par rapport aux variables d'espace (coordonnées selon X, Z dans l'exemple considéré), c'est-à-dire :

$$\Delta\varepsilon = \begin{bmatrix} \dfrac{du_x}{dx} \\ \dfrac{du_z}{dz} \end{bmatrix}$$

Eventuellement, selon un exemple de mise en oeuvre, seule la déformation/déformation partielle uniaxiale en direction Z est calculée. En effet, la compression liée à la pression externe de la sonde est principalement dans cette direction. De la même manière, dans un calcul suivant de contrainte, seul la contrainte/contrainte partielle uniaxiale dans la direction Z est éventuellement calculée. On simplifie ainsi les calculs.

Comme pour les vecteurs déplacements, on peut construire une image de déformation partielle ($I\Delta\varepsilon_k$) à partir de l'ensemble des déformations partielles calculées en tout point de coordonnées (x, z) du plan X-Z (plan d'image).

Ces vecteurs de déformations partielles ou images de déformation partielle ($I\Delta\varepsilon_k$) peuvent être visualisés, notamment au moyen de l'écran 4a du micro-ordinateur, sous la forme d'un film ralenti.

## Détermination du mouvement du milieu par la pression externe

La déformation du milieu 2 liée aux variations de la pression externe P exercée sur la surface extérieure 3 est une déformation lente, élastique quasi-uniforme. Cette déformation se propage à l'intérieur du milieu viscoélastique 2, et provoque le déplacement des particules 5 et des éléments inclus dans le milieu 2.

Ce déplacement est néfaste à l'analyse des caractéristiques des divers points de l'image du milieu, et notamment l'évaluation de la déformation en ces points et/ou de l'élasticité de ces points.

La présente divulgation considère donc de corriger des images pour compenser ces mouvements, ce qui permet de conserver les particules et/ou éléments internes du milieu 2 à des emplacements quasi constants dans l'image, permettant ainsi d'en étudier les caractéristiques plus précisément.

La déformation externe provoquée par la pression externe P peut être considérée comme provoquant un mouvement entre une image du milieu d'indice k ($I_k$) et une image du milieu précédente d'indice k-1 ($I_{k-1}$).

Le mouvement peut alors être déterminé par le champ de déplacement ($u_k$) entre une image du milieu d'indice k et une image du milieu précédente d'indice k-1.

Notamment, ce mouvement peut être modélisé par une transformation géométrique $T_k$ d'indice k qui représente d'une manière générale et de manière simple l'ensemble des déplacements du champ de déplacement à l'étape d'indice d'image k, $u_k(x, z)$, c'est-à-dire avec un nombre de paramètres très réduit, et par exemple inférieur à dix.

Alors, une compensation de tout type d'images à partir des mouvements précédents peut être effectué par application des transformations géométriques précédentes, c'est-à-dire par application des transformations géométriques $T_i$, l'indice i variant de 1 à k.

Selon une première variante, la transformation géométrique $T_k$ comprend une translation, comme cela est représenté entre la figure 2A et la figure 2B.

Selon une seconde variante, la transformation géométrique $T_k$ comprend une translation et une homothétie.

Selon une seconde variante, la transformation géométrique $T_k$ comprend une translation, une homothétie et une rotation.

Ainsi, dans le plan X-Z, plan d'image, la transformation géométrique peut être mise sous une forme matricielle du type :

$$T_k = \begin{bmatrix} Hx.\cos\theta & -Hz.\sin\theta & Tx \\ Hx.\sin\theta & Hz.\cos\theta & Tz \\ 0 & 0 & 1 \end{bmatrix}$$

avec les paramètres de la transformation géométrique (Tk) suivants :

Tx, Ty coefficients de translation dans le plan d'image,
Hx, Hz coefficient d'homothétie le plan d'image, et
$\theta$ angle de rotation d'axe perpendiculaire au plan d'image.

Ainsi, si l'on prend trois points P1, P2, et P3 de l'image de cordonnées respectives (x1,z1), (x2,z2), (x3,z3), on a les relations suivantes :

$$A = \begin{bmatrix} x1 & x2 & x3 \\ z1 & z2 & z3 \\ 1 & 1 & 1 \end{bmatrix}$$

$$B = \begin{bmatrix} x1 + dx1 & x2 + dx2 & x3 + dx3 \\ z1 + dz1 & z2 + dz2 & z3 + dz3 \\ 0 & 0 & 0 \end{bmatrix}$$

avec

$$dx1 = u_{x1} \qquad dz1 = u_{z1}$$

$$dx2 = u_{x2} \qquad dz2 = u_{z2}$$

$$dx3 = u_{x3} \qquad dz3 = u_{z3}$$

pour reprendre les notation des composantes X et Z des vecteurs déplacements des points P1, P2 et P3.
En outre, on a une relation permettant de calculer la transformation géométrique $T_k$ à partir des matrices A et B précédentes, c'est-à-dire une matrice A correspondant aux cordonnées des trois points dans l'image, et une matrice B correspondant aux coordonnées de ces mêmes trois points avec les déplacements de ces points (le mouvement). Ainsi, la matrice de la transformation géométrique est obtenue par le produit matriciel de la matrice B et de l'inverse de la matrice A :

$$T_k = B.A^{-1}$$

L'application de cette relation permet de calculer la transformation géométrique $T_k$ à partir de trois point P1, P2, P3.
Inversement, une fois la transformation géométrique $T_k$ établie, cette relation permet de connaître les coordonnées (x, z) de tout point P par la relation inverse :

$$B = T_k.A$$

Alors, une compensation de tout type d'images à partir des mouvements précédents peut être effectué par multiplication matricielle des transformations géométriques précédentes, c'est-à-dire par multiplication matricielle des transformations géométriques $T_i$, l'indice i variant de 1 à k.

Les relations précédentes établies avec 3 points peuvent être généralisées à un groupe de points de l'image, le groupe de points comprenant 3, 4, 5 ou 6 points de l'image. Le groupe de points comprend entre trois et dix points de l'image.

En outre, dans une variante, on prendra avantageusement une population de trois points (de groupe de points) de l'image, ladite population comprenant un nombre Ng de groupes de points de grande valeur. Par exemple, la taille de cette population, le nombre Ng de groupes de points est supérieur à cent.

Alors, les paramètres de la transformation géométrique $T_k$ sont obtenus par des valeurs médianes des paramètres calculés à partir de la population des groupes de points de l'image.

Notamment, cette technique peut être appliquée à une image du champ de déplacement $u_k$ pour en déduire le mouvement entre une image du milieu d'indice k et une image du milieu précédente d'indice k-1.

Grâce à l'utilisation d'une population de groupes de points, on peut déterminer une transformation géométrique qui représente de manière plus globale le mouvement entre l'image du milieu d'indice k et une image du milieu précédente d'indice k-1

Avantageusement, la sélection des points dans chaque groupe se fait de manière aléatoire dans l'image. Grâce à cette disposition, on peut déterminer une transformation géométrique qui représente de manière plus globale et plus fiable le mouvement entre images.

**Détermination d'images de déformation partielle corrigée I$\Delta\varepsilon_k$***

Les images de déformation partielle I$\Delta\varepsilon_k$ sont alors corrigées pour ramener ces images dans une situation conforme à la première image du milieu $I_0$, supprimant ainsi les mouvements provoqués par la déformation de la pression externe P dans ces images de déformation partielles I$\Delta\varepsilon_k$.

Cette correction est effectuée en utilisant à chaque image de déformation partielle I$\Delta\varepsilon_k$ d'indice d'image k, l'ensemble des mouvements global déterminés entre chaque image du milieu. Plus précisément, il faut en appliquer les mouvements inverses pour revenir dans la situation de la première image $I_0$.

Autrement dit, les images de déformation partielle corrigées I$\Delta\varepsilon_k$* contiennent des déformations partielles corrigées $\Delta\varepsilon_k$* et elles sont calculées par compensation desdites images de déformation partielle I$\Delta\varepsilon_k$ à partir des mouvements associés aux images du milieu d'indices compris entre 1 et k inclus.

**Détermination d'une image de déformation corrigée I$\varepsilon_k$***

La déformation $\varepsilon_k(x,z)$ est obtenue en cumulant les déformations partielles successives, c'est-à-dire :

$$\varepsilon_k(x,z) = \sum_{i=1}^{k} \Delta\varepsilon_i(x,z)$$

Dans le cas présent, la déformation $\varepsilon_k(x,z)$ (déformation corrigée) est obtenue en cumulant les déformations partielles successives corrigées $\Delta\varepsilon_k$* :

$$\varepsilon_k^*(x,z) = \sum_{i=1}^{k} \Delta\varepsilon_i^*(x,z)$$

On peut construire une image de déformation corrigée (I$\Delta\varepsilon_k$*) à partir de l'ensemble des déformations corrigées $\varepsilon_k(x,z)$ calculées en tout point de coordonnées (x, z) du plan X-Z (plan d'image).

Ces images de déformation corrigées I$\varepsilon_k$* peuvent être visualisés sous la forme d'un film ralenti.

**Procédé pour déterminer une image corrigée**

En résumé, selon une première forme de réalisation du procédé représenté en **figure 3** selon la présente divulgation, le procédé mis en oeuvre comprend les étapes suivantes :

(d1) la détermination d'une succession temporelle d'images du milieu ($I_k$), k étant un indice d'image compris entre 0 et N, et la première image de ladite succession étant supposée sans déformation,

(d2) la détermination du mouvement pour chaque image du milieu d'indice k entre 1 et N, le mouvement étant entre une image du milieu d'indice k ($I_k$) et l'image du milieu précédente d'indice k-1 ($I_{k-1}$),

(d3) la détermination d'une succession temporelle d'images de déformation partielle ($I\Delta\varepsilon_k$) à partir de l'image du milieu d'indice k ($I_k$) et l'image du milieu précédente d'indice k-1 ($I_{k-1}$),

(d4) la détermination d'images de déformation partielle corrigées ($I\Delta\varepsilon_k$*) par compensation desdites images de déformation partielle ($I\Delta\varepsilon_k$) à partir des mouvements précédents, c'est-à-dire les mouvements associés aux images du milieu d'indices compris entre 1 et k inclus,

(d5) la détermination d'une image de déformation corrigée ($I\varepsilon_n$*) par une somme ou cumul des images de déformation partielle corrigées ($I\Delta\varepsilon_n$*) des indices compris entre 1 et k inclus.

Par somme ou cumul d'images, on comprend que les valeurs de pixels de même coordonnée spatiale desdites images sont sommées (i.e ajoutées les unes aux autres) pour former la valeur du pixel correspondant de l'image résultante.

Grâce à ces dispositions, on obtient une image de déformation corrigée $I\varepsilon_n$* du milieu 2 qui n'est pas ou peu influencée par les déformation et mouvements induits par la pression externe P, ce qui permet d'obtenir des valeurs de déformation plus exacte et plus précises que dans l'art antérieur.

## Détermination d'une image d'élasticité du milieu $IE_k$

Le document US 2005/252295 propose une technique d'imagerie d'élasticité du milieu viscoélastique 2 : Le dispositif d'imagerie 1 étudie la propagation des ondes élastiques de cisaillement dans ce milieu. Les mouvements des ondes élastiques de cisaillement sont suivis par les moyens décrits précédemment, et notamment par le micro-ordinateur 4.

On procède en plusieurs étapes :

(e1.1) une étape d'excitation au cours de laquelle le micro-ordinateur 4 fait générer une onde élastique de cisaillement dans le milieu viscoélastique 2, en faisant émettre au moins une onde ultrasonore focalisée dans le milieu viscoélastique par la sonde 6,

(e1.2) une étape d'observation au cours de laquelle on observe la propagation de l'onde de cisaillement simultanément en une multitude de points du champ d'observation dans le milieu viscoélastique 2, cette étape comprenant des sous-étapes durant lesquelles on génère une pluralité d'images intermédiaires du milieu $II_{j,k}$ temporellement successives, j étant un indice d'image intermédiaire compris entre 0 et M inclus, M+1 étant le nombre d'images intermédiaires produite.

Chaque image intermédiaire du milieu est par exemple générée par le procédé de détermination d'une image du milieu $I_k$ précédemment décrit, dans lequel on fait émettre au moins un tir d'onde ultrasonore de compression non focalisée par la sonde 6, et on détecte et enregistre par la sonde 6 les signaux acoustiques reçus, et on traite ces signaux acoustiques pour construire une image intermédiaire du milieu ($II_{j,k}$).

La focalisation et la chronologie de l'onde ultrasonore focalisée émise à l'étape (e1), ainsi que la chronologie des ondes ultrasonores non focalisées émises à l'étape (e2), sont adaptées pour que des ondes ultrasonores non focalisées parviennent dans le champ d'observation lors de la propagation de l'onde de cisaillement dans ce champ d'observation. Ainsi, l'onde de cisaillement est visible dans les images intermédiaires du milieu $II_{j,k}$ générées.

On procède alors à :

(e1.3) une étape de traitement des images intermédiaires du milieu pour déterminer une image d'élasticité du milieu $IE_k$.

Les images intermédiaires du milieu $II_{j,k}$ sont traitées pour calculer un champ de déplacement $u_{j,k}$, par exemple par corrélation ou intercorrélation comme précédemment. Par exemple par intercorrélation entre une image intermédiaire du milieu d'indice j ($II_{j,k}$) et l'image intermédiaire du milieu du milieu d'indice j-1 ($I_{j-1,k}$). Les vecteurs de déplacement déterminés sont utilisables pour visualiser la propagation de l'onde de cisaillement sous la forme d'un film. On calcule éventuellement en outre les déformations partielles $\Delta\varepsilon$ en chaque point du milieu 2 à partir du champ de déplacement.

A partir des champs de déplacements ou de déformations, le micro-ordinateur 4 peut avantageusement procéder ensuite à une étape de cartographie au cours de laquelle, à partir de l'évolution du paramètre de mouvement (déplacement ou déformation partielle) au cours du temps dans le champ d'observation, on calcule au moins un paramètre de propagation de l'onde de cisaillement, soit en certains points du champ d'observation choisis, soit dans tout le champ d'observation.

On peut alors construire une image d'élasticité $IE_k$, correspondant à l'ensemble des paramètres des de propagation de l'onde de cisaillement aux divers points du champ d'observation.

Cette image d'élasticité IE$_k$ du milieu peut être visualisée, notamment au moyen de l'écran 4a du micro-ordinateur où la valeur du paramètre de propagation est illustrée par un paramètre optique tel que par un niveau de gris ou par un niveau chromatique.

Le paramètre de propagation de l'onde de cisaillement qui est calculé au cours de l'étape de cartographie est choisi par exemple parmi :

- la vitesse Cs des ondes de cisaillement, ou
- le module de cisaillement $\mu$, ou
- le module d'Young E=$3\mu$, ou
- l'atténuation $\alpha$ des ondes de cisaillement, ou
- l'élasticité du cisaillement $\mu$1, ou
- la viscosité de cisaillement $\mu$2, ou
- le temps de relaxation mécanique is des tissus du milieu.

Par exemple, on peut calculer en différents points du champ d'observation :

- la valeur de la célérité Cs de l'onde de cisaillement, qui donne accès à la dureté des tissus,
- la valeur du temps de relaxation mécanique $\tau$s des tissus, caractéristiques de la viscosité locale du milieu.

Pour cela, on utilise un modèle de propagation de l'onde de cisaillement, par exemple représenté par l'équation de propagation suivante, à laquelle obéissent les déplacements u engendrés par les ondes de cisaillement en chaque position r du milieu :

$$\rho \frac{\partial^2 \vec{u}(\vec{r},t)}{\partial t^2} = c_s^{\ 2}\,(1+\tau_s \frac{\partial \cdot}{\partial t}).\vec{\nabla}^2 \vec{u}(\vec{r},t)$$

Où

$\rho$ est la densité des tissus,

$\tau$S est le temps de relaxation mécanique des tissus, et

cS est la célérité de l'onde de cisaillement, directement reliée au module d'Young E des tissus par la relation :

$$c_s = \sqrt{\frac{E}{3\rho}}$$

La résolution de cette équation de propagation avec l'ensemble des déplacements u permet d'obtenir les paramètres de propagation (cS, $\tau$S) cités ci-dessus.

Des variantes de calcul du/des paramètre(s) de propagation sont possibles. Notamment, on peut utiliser l'équation d'onde dans le domaine de Fourier, en moyennant par exemple les valeurs sur une bande de fréquences. On peut aussi utiliser les déformations partielles à la place des déplacements.

On peut également établir des cartographies des paramètres de propagation, i.e. des images d'élasticité, avec des ondes de cisaillement différentes. Il est alors possible de les combiner, par exemple en les moyennant, de façon à obtenir une cartographie plus précise.

La vitesse de propagation de l'onde de cisaillement dans le milieu 2 est suffisamment grande pour considérer qu'il n'y a pas de variation de la pression externe P exercée sur la surface externe 3 pendant ce processus durant lequel on prend une pluralité d'images du milieu (images intermédiaires) destinées à déterminer une image d'élasticité IE (paramètre de propagation de l'onde de cisaillement). Ainsi, ces images du milieu ne sont pas corrigées par les mouvements du milieu.

Par contre, l'image d'élasticité du milieu IE$_k$ doit être corrigée avec le mouvement à l'instant temporel considéré ou à un instant temporel proche (représenté par l'indice k) pour pouvoir être comparée à l'image du milieu initial I$_0$, ou pour pouvoir être comparée à l'image de déformation corrigée I$\varepsilon_k$*.

### Détermination d'une image d'élasticité corrigée IE$_k$*

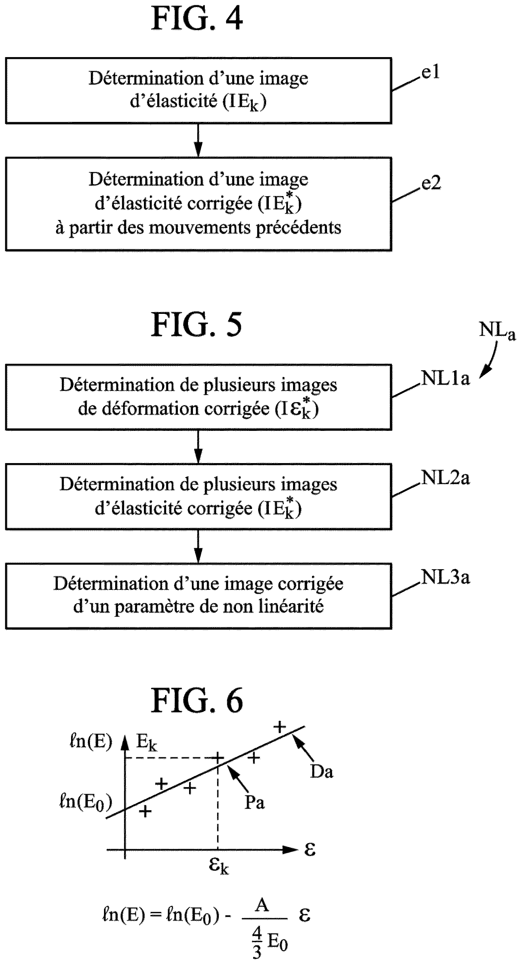

L'image d'élasticité IE$_k$ est alors corrigée dans un procédé représenté en **figure 4**, dans lequel on cherche à ramener cette image d'élasticité IE$_k$ dans une situation conforme à la première image du milieu I$_0$ pour supprimer les mouvements provoqués par la déformation de la pression externe P.

Cette correction est effectuée en utilisant à chaque image d'élasticité IE$_k$ d'indice d'image k, l'ensemble des mouvements globaux déterminés entre chaque image du milieu. Plus précisément, il faut en appliquer les mouvements inverses pour revenir dans la situation de la première image I$_0$.

Autrement dit, le procédé comprend les étapes suivantes :

(e1) détermination d'une image d'élasticité (IE$_k$),

(e2) la détermination d'une image d'élasticité corrigée (IE$_k$*) par compensation de l'image d'élasticité (IE$_k$) à partir des mouvements précédents associés aux images du milieu d'indices compris entre 1 et k inclus.

### Détermination d'une image d'un **paramètre** de **non linéarité INL$_k$**

Dans un domaine élastique linéaire, le milieu 2 se déforme proportionnellement à la contrainte σ et suit la loi de Hooke :

$$\sigma = E_0 . \varepsilon$$

où

$E_0$ est le module d'Young du domaine linéaire, et
$\varepsilon$ est la déformation.

Dans un domaine élastique non linéaire, cette proportionnalité n'est plus valable. Dans la plupart de cas, le module d'Young E du matériau du milieu 2 augmente avec la compression.

On définit alors le module d'élasticité ou module d'Young E comme étant la pente de la courbe contrainte-déformation c'est-à-dire :

$$E = \frac{\Delta\sigma}{\Delta\varepsilon}$$

(eq. 1)

où

E est le module d'Young,
$\Delta\sigma$ est la variation de contrainte locale, c'est-à-dire la contrainte partielle,
$\Delta\varepsilon$ est la variation de déformation locale, c'est-à-dire la déformation partielle.

Un paramètre de non linéarité de l'élasticité peut par exemple être le coefficient de Landau appelé le module d'élasticité en cisaillement du troisième ordre A mentionné dans les documents :

- « Acoustoelasticity in soft solids : Assesment of the non-linear shear modulus with the acoustic radiation force », Gennisson et Al., J. Acoust. Soc. Am (122), December 2007, p. 3211-3219, et
- « Quantitative Imaging of Nonlinear Shear Modulus by Combining Static Elastography and Shear Wave Elastography », H. Latorre-Ossa et Al., IEEE Transactions on Ultrasonics, Ferroelectrics, and Frequency Control, Vol. 51, no. 4, p. 833-839.

Notamment, l'équation (1) de ce dernier document peut être réécrite sous la forme d'une relation (R1) entre l'élasticité (module d'Young) E et la contrainte σ :

$$E = E_0 - \frac{A}{\frac{4}{3} \cdot E_0} \cdot \sigma$$

(eq. 2)

où

E est le module d'Young ou module d'élasticité,

$E_0$ est le module d'Young du domaine linéaire, c'est-à-dire le module d'Young du matériau du milieu sans compression, donc la première image d'élasticité corrigée dans la succession temporelle des images,

$\sigma$ est la contrainte et par exemple la contrainte $\sigma_z$ uniaxiale dans la direction Z, verticale, correspondant sensiblement à la compression induite par la pression exercée externe par l'utilisateur de la sonde 6, et

A est le paramètre de non linéarité recherché.

Par différentiation de la relation R1 précédente, on obtient :

$$\Delta E = - \frac{A}{\frac{4}{3} \cdot E_0} \cdot \Delta \sigma$$

En divisant par la définition du module d'Young de l'équation (eq. 1) et par intégration on obtient une relation R2 entre l'élasticité (module d'Young) E et la déformation $\varepsilon$, soit :

$$\ln(E) = \ln(E_0) - \frac{A}{\frac{4}{3} \cdot E_0} \cdot \varepsilon$$

(eq. 3)

où

E est le module d'Young,

$E_0$ est le module d'Young du domaine linéaire, c'est-à-dire le module d'Young du matériau du milieu sans compression, donc la première image d'élasticité corrigée dans la succession temporelle des images,

ln() est la fonction de logarithme népérien,

$\varepsilon$ est la déformation, et

A est le paramètre de non linéarité à déterminer.

Selon une première variante du procédé NLa, représenté en **figure 5**, basé sur des images de déformation et des images d'élasticité, le procédé mis en oeuvre par le micro-ordinateur 4 comprend en outre les étapes suivantes :

(NL1a) la détermination d'une pluralité d'images de déformation corrigée ($I\varepsilon_k{}^*$), comme présenté précédemment,
(NL2a) la détermination d'une pluralité d'images d'élasticité corrigée ($IE_k{}^*$), comme présenté précédemment, lesdites images de déformation corrigé et lesdites images d'élasticité étant temporellement entrelacées, et
(NL3a) la détermination d'une image d'un paramètre de non linéarité ($INL_k$) à partir de la pluralité d'images de déformation corrigée ($I\varepsilon_k{}^*$) et de la pluralité d'images d'élasticité corrigée ($IE_k{}^*$).

En fait, un paramètre de non linéarité d'un pixel de l'image est déterminé par l'ensemble (la pluralité) des valeurs de déformation corrigée et d'élasticité corrigée de ce même pixel.

Par exemple, ce paramètre de non linéarité est déterminé par une régression linéaire de cette pluralité de couples de valeurs. Nous appellerons cette régression linéaire, une régression linéaire en déformation.

Chaque couple de valeurs est formé pour un pixel de l'image et comprend :

- une première valeur de ce même pixel d'une image de déformation corrigée, et
- une deuxième valeur de ce même pixel d'une image de d'élasticité corrigée,

les images de déformation corrigée et d'élasticité considérées étant des images temporellement suspensives ou temporellement proches. Ces images peuvent présenter des indices d'image identiques ou proche.

Eventuellement, les images de déformation et les images d'élasticité peuvent avoir des indices d'image différents et il est nécessaire de prendre des images d'indices correspondant à des instants temporels successifs ou temporellement proches comme explicités ci-dessus, c'est-à-dire que l'on effectue une correspondance entre le temps et l'indice de chaque image considérée.

La **figure 6** est une représentation pour un pixel d'image d'une pluralité de points Pa, chaque point Pa correspondant à un tel couple de valeurs ($E_k$, $\varepsilon_k$). Tous ces points Pa sont sensiblement alignés selon une droite Da dans un graphique ayant pour abscisse la valeur de déformation corrigée $\varepsilon$ (pour ce pixel) et ayant pour ordonnée le logarithme népérien de la valeur du module d'Young E (pour ce même pixel de l'image).

La régression linéaire en déformation utilise alors la relation R2 entre l'élasticité (module d'Young) E et la déformation $\varepsilon$ (eq. 3) :

$$\ln(E) = \ln(E_0) - \frac{A}{\frac{4}{3} \cdot E_0} \cdot \varepsilon$$

où plus précisément dans le cas présent d'application :

E est le module d'Young du pixel de l'image d'élasticité corrigée considérée,
$E_0$ est le module d'Young du pixel de la première image d'élasticité corrigée,
ln() est la fonction de logarithme népérien,
$\varepsilon$ est la déformation du pixel de l'image de déformation corrigée considérée, et
A est le paramètre de non linéarité déterminé dudit pixel par ladite régression linéaire en déformation.

La résolution de cette régression linéaire en déformation pour un ensemble de couples de valeurs extraits des pluralités d'images de déformation corrigée ($I\varepsilon_k{}^*$) et d'images d'élasticité corrigée ($IE_k{}^*$) donne les valeurs du paramètre de non linéarité A de chaque pixel de l'image.

Grâce à ces calculs, on obtient une image d'un paramètre de non linéarité $INL_k$ du milieu 2 qui utilise des images corrigées. Cette image ne présente pas les artéfacts d'images non corrigées de l'art antérieur. Cette image n'est pas ou peu influencée par les déformations et mouvements induits par la pression externe P. Les valeurs de paramètre de non linéarité A obtenues sont plus exactes et plus précises qu'avec les méthodes de l'art antérieur.

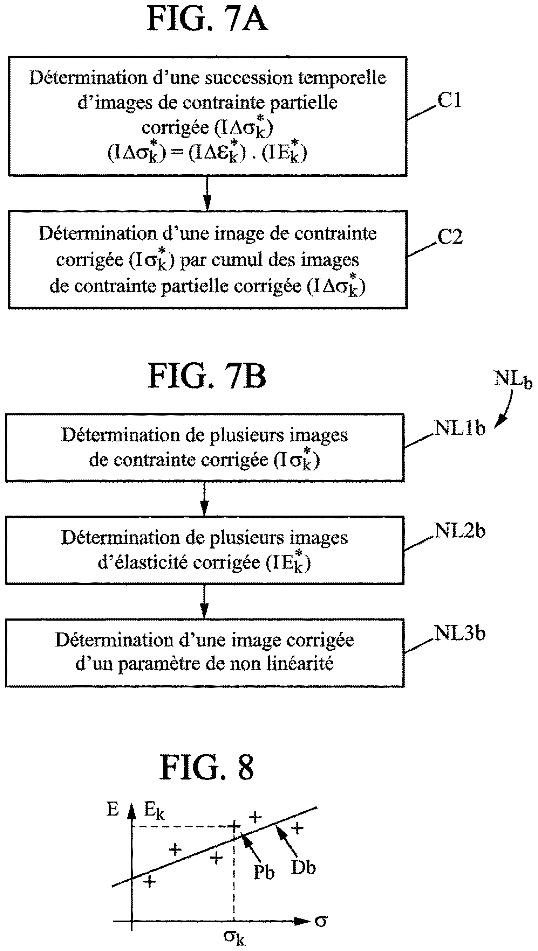

Selon une seconde variante du procédé NLb, représenté en **figure 7A et 7B**, basé sur des images de contrainte et des images d'élasticité, le procédé mis en oeuvre par le micro-ordinateur 4 comprend :

- une première phase durant laquelle des images de déformation partielles sont converties en images de contrainte corrigées, et
- une deuxième phase durant laquelle une image d'un paramètre de non linéarité est déterminée.

Durant la première phase représentée en **figure 7A**, on effectue :

(c1) la détermination d'une succession temporelle d'images de contrainte partielle corrigée ($I\Delta\sigma_k{}^*$) par une multiplication de l'image de déformation partielle corrigée ($I\Delta\varepsilon_k{}^*$) d'indice k et de l'image d'élasticité corrigée ($IE_k{}^*$) d'indice k, et
(c2) la détermination d'une image de contrainte corrigée $I\sigma_k{}^*$) par une somme des images de déformation partielle corrigée ($I\Delta\sigma_k{}^*$) des indices entre 1 et k inclus.

Par multiplication d'images, on comprend ici que les valeurs de pixels de même coordonnée spatiale desdites images sont multipliées (i.e. multipliée l'une avec l'autre) pour former la valeur du pixel correspondant de l'image résultante. Ici, on multiplie donc une valeur de déformation partielle corrigée $\Delta\varepsilon^*$, calculée par les étapes du procédé explicité précédemment et illustré en figure 3, par une valeur d'élasticité corrigée E* (module d'Young corrigé), calculée par les étapes du procédé explicité précédemment et illustré en figure 4, pour obtenir la valeur de contrainte corrigée $\Delta\sigma^*$ dudit pixel.

Ainsi, la contrainte partielle est calculée en chaque pixel de l'image par :

$$\Delta\sigma^* = E^* . \Delta\varepsilon^*$$

Par somme ou cumul d'images, on comprend que les valeurs de pixels de même coordonnée spatiale desdites images sont sommées (i.e ajoutées les unes aux autres) pour former la valeur du pixel correspondant de l'image résultante.

C'est-à-dire que :

$$\sigma_k^*(x,z) = \sum_{i=1}^{k} \Delta\sigma_i^*(x,z)$$

Cette formule est appliquée en tout pixel de l'image pour construire l'image de contrainte corrigée($I\sigma_k^*$).

Durant la deuxième phase représentée en **figure 7B**, on effectue :

(NLb1) la détermination d'une pluralité d'images de contrainte corrigée ($I\sigma_k^*$),
(NLb2) la détermination d'une pluralité d'images d'élasticité corrigée ($IE_k^*$), lesdites images de déformation corrigé et lesdites images d'élasticité étant temporellement entrelacées, et
(NLb3) la détermination d'une image d'un paramètre de non linéarité ($INL_k$) à partir de la pluralité d'images de contrainte corrigée ($I\varepsilon_k^*$) et de la pluralité d'images d'élasticité corrigée ($IE_k^*$).

En fait, un paramètre de non linéarité d'un pixel de l'image est déterminé par l'ensemble (la pluralité) des valeurs de contrainte corrigée et d'élasticité corrigée de ce même pixel.

Par exemple, ce paramètre de non linéarité est déterminé par une régression linéaire de cette pluralité de couples de valeurs. Nous appellerons cette régression linéaire, une régression linéaire en contrainte.

Chaque couple de valeurs est formé pour un pixel de l'image et comprend :

- une première valeur de ce même pixel d'une image de contrainte corrigée, et
- une deuxième valeur de ce même pixel d'une image de d'élasticité corrigée,

les images de contrainte corrigée et d'élasticité considérées étant des images temporellement suspensives ou temporellement proches. Ces images peuvent présenter des indices d'image identiques ou proche.

Eventuellement, les images de contrainte et les images d'élasticité peuvent avoir des indices d'image différents et il est nécessaire de prendre des images d'indices correspondant à des instants temporels successifs ou temporellement proches comme explicités ci-dessus, c'est-à-dire que l'on effectue une correspondance entre le temps et l'indice de chaque image considérée.

La **figure 8** est une représentation pour un pixel d'image d'une pluralité de points Pb, chaque point Pb correspondant à un couple de valeurs ($E_k$, $\sigma_k$). Tous ces points Pb sont sensiblement alignés selon une droite Db dans un graphique ayant pour abscisse la valeur de contrainte corrigée $\sigma$ (pour ce pixel) et ayant pour ordonnée la valeur du module d'Young E (pour ce même pixel de l'image).

La régression linéaire en contrainte utilise alors la relation R1 entre l'élasticité (module d'Young) E et la contrainte $\sigma$ (eq. 2) :

$$E = E_0 - \frac{A}{\frac{4}{3} \cdot E_0} \cdot \sigma$$

où plus précisément dans le cas présent d'application :

E est le module d'Young du pixel de l'image d'élasticité corrigée considérée,
$E_0$ est le module d'Young du pixel de la première image d'élasticité corrigée,
ln() est la fonction de logarithme népérien,
$\varepsilon$ est la déformation du pixel de l'image de déformation corrigée considérée, et
A est le paramètre de non linéarité déterminé dudit pixel par ladite régression en contrainte.

La résolution de cette régression linéaire en contrainte pour un ensemble de couples de valeurs extraites des pluralités d'images de contrainte corrigée ($I\sigma_k^*$) et d'images d'élasticité corrigée ($IE_k^*$) donne les valeurs du paramètre de non linéarité A de chaque pixel de l'image.

Grâce à ces calculs, on obtient une image d'un paramètre de non linéarité $INL_k$ du milieu 2 qui utilise des images corrigées. Cette image ne présente pas les artéfacts d'images non corrigées de l'art antérieur. Cette image n'est pas ou peu influencée par les déformations et mouvements induits par la pression externe P. On obtient des valeurs de paramètre de non linéarité A plus exacts et plus précis que dans l'art antérieur.

**Revendications**

1. **Procédé pour déterminer par ultrasons une image corrigée** d'un milieu, le procédé comprenant :

   - la détermination d'une succession temporelle d'images du milieu ($I_k$), k étant un indice d'image compris entre 0 et N, et la première image de ladite succession étant supposée sans déformation,
   - la détermination du mouvement pour chaque image du milieu d'indice k entre 1 et N, le mouvement étant entre une image du milieu d'indice k ($I_k$) et l'image du milieu précédente d'indice k-1 ($I_{k-1}$),
   **caractérisé en ce que** ledit procédé comprend en outre
   - la détermination d'une succession temporelle d'images de déformation partielle ($I\Delta\varepsilon_k$) à partir de l'image du milieu d'indice k ($I_k$) et l'image du milieu précédente d'indice k-1 ($I_{k-1}$),
   - la détermination d'images de déformation partielle corrigée ($I\Delta\varepsilon_k*$) par compensation desdites images de déformation partielle ($I\Delta\varepsilon_k$) à partir des mouvements précédents, c'est-à-dire les mouvements associés aux images du milieu d'indices compris entre 1 et k inclus, et
   - la détermination d'une image de déformation corrigée ($I\varepsilon_k*$) par une somme des images de déformation partielle corrigée ($I\Delta\varepsilon_k*$) des indices entre 1 et k inclus, et

   dans lequel la détermination du mouvement à l'indice k est effectuée par :

   - détermination d'un champ de déplacement ($u_k$) entre une image du milieu ($I_k$) et l'image du milieu précédente ($I_{k-1}$),
   - détermination d'une transformation géométrique ($T_k$) d'image à partir dudit champ de déplacement ($u_k$), ladite transformation géométrique représentant le champ de déplacement ($u_k$) avec un nombre de paramètres inférieur à dix.

2. Procédé selon la revendication 1, dans lequel le champ de déplacement est calculé par intercorrélation entre une image du milieu (Ik) et l'image du milieu précédente (Ik-1), ou par un algorithme de suivi de sous-images entre une image du milieu (Ik) et l'image du milieu précédente (Ik-1).

3. Procédé selon la revendication 2, dans lequel l'algorithme de suivi est un algorithme de Lucas-Kanade.

4. Procédé selon l'une des revendications 1 à 3, dans lequel la transformation géométrique (Tk) comprend au moins une translation, ou une translation et une homothétie, ou une translation, une homothétie et une rotation.

5. Procédé selon l'une des revendications 1 à 3, dans lequel la transformation géométrique (Tk) comprend une translation, une homothétie et une rotation, et dans lequel la transformation géométrique est mise sous une forme matricielle telle que :

$$T_k = \begin{bmatrix} Hx.cos\theta & -Hz.sin\theta & Tx \\ Hx.sin\theta & Hz.cos\theta & Tz \\ 0 & 0 & 1 \end{bmatrix}$$

avec les paramètres de la transformation géométrique (Tk) dans le plan d'image suivants :

   Tx, Ty coefficients de translation,
   Hx, Hz coefficient d'homothétie, et
   $\theta$ angle de rotation d'axe perpendiculaire au plan d'image.

6. Procédé selon la revendication 1, dans lequel des paramètres de la transformation géométrique (Tk) sont obtenus par des valeurs médianes d'une population de paramètres calculés à partir de groupes de points du champ de déplacement (uk).

**7.** Procédé selon la revendication 6, dans lequel :

- un groupe comprend entre trois et dix points du champ de déplacement ($u_k$), et
- la population est supérieure à cent groupes.

**8.** Procédé selon la revendication 1, comprenant en outre :

(e1) la détermination d'une image d'élasticité ($IE_k$) du milieu, et
(e2) la détermination d'une image d'élasticité corrigée ($IE_k^*$) par compensation de ladite image d'élasticité ($IE_k$) à partir des mouvements précédents, c'est-à-dire les mouvements associés aux images du milieu d'indices compris entre 1 et k inclus.

**9.** Procédé selon la revendication 8, dans lequel l'image d'élasticité (IEk) produite à l'étape (e1) est générée par les sous-étapes suivantes :

(e1.1) une étape d'excitation au cours de laquelle on génère une onde de cisaillement dans le milieu, en faisant émettre au moins une onde ultrasonore focalisée,
(e1.2) une étape d'observation au cours de laquelle on observe la propagation de l'onde de cisaillement en déterminant une succession temporelle d'images intermédiaires du milieu ($II_{j,k}$), j étant un indice d'image intermédiaire compris entre 0 et M,
(e1.3) une étape de traitement au cours de laquelle on détermine l'image d'élasticité ($IE_k$) à partir desdites images intermédiaires du milieu ($II_{j,k}$) et d'un modèle de propagation d'onde de cisaillement.

**10.** Procédé selon la revendication 8, dans lequel on effectue en outre :

- la détermination d'une pluralité d'images de déformation corrigée ($I\varepsilon_k^*$),
- la détermination d'une pluralité d'images d'élasticité corrigée ($IE_k^*$), lesdites images de déformation corrigé et lesdites images d'élasticité étant temporellement entrelacées, et
- la détermination d'une image d'un paramètre de non linéarité ($INL_k$) à partir de la pluralité d'images de déformation corrigée ($I\varepsilon_k^*$) et de la pluralité d'images d'élasticité corrigée ($IE_k^*$).

**11.** Procédé selon la revendication 10, dans lequel la valeur de chaque pixel de l'image d'un paramètre de non linéarité ($INL_k$) est déterminée par régression linéaire en déformation de couples de valeurs, la première valeur de chaque couple correspondant à la valeur d'un même pixel d'une image de déformation corrigée de la pluralité et la deuxième valeur du couple correspondant à la valeur d'un même pixel d'une image d'élasticité de la pluralité, ladite image de déformation corrigée de la pluralité et ladite image d'élasticité corrigée de la pluralité étant temporellement successives ou proches, et

dans lequel la régression linéaire en déformation est établie sur la base de la relation entre la déformation et l'élasticité suivante :

$$\ln(E) = \ln(E_0) - \frac{A}{\frac{4}{3} \cdot E_0} \cdot \varepsilon$$

où

E est le module d'Young du pixel de l'image d'élasticité corrigée considérée,
$E_0$ est le module d'Young du pixel de la première image d'élasticité corrigée,
ln() est la fonction de logarithme népérien,
$\varepsilon$ est la déformation du pixel de l'image de déformation corrigée considérée, et
A est le paramètre de non linéarité déterminé dudit pixel par ladite régression linéaire en déformation.

**12.** Procédé selon la revendication 8, dans lequel on effectue en outre :

- la détermination d'une succession temporelle d'images de contrainte partielle corrigée ($I\Delta\sigma_k{}^*$) par une multiplication de l'image de déformation partielle corrigée ($I\Delta\varepsilon_k{}^*$) d'indice k et de l'image d'élasticité corrigée ($IE_k{}^*$) d'indice k,
- la détermination d'une image de contrainte corrigée ($I\sigma_k{}^*$) par une somme des images de déformation partielle corrigée ($I\Delta\sigma_k{}^*$) des indices entre 1 et k inclus.

**13.** Procédé selon la revendication 12, dans lequel on effectue en outre :

- la détermination d'une pluralité d'images de contrainte corrigée ($I\sigma_k{}^*$),
- la détermination d'une pluralité d'images d'élasticité corrigée ($IE_k{}^*$), lesdites images de déformation corrigé et lesdites images d'élasticité étant temporellement entrelacées, et
- la détermination d'une image d'un paramètre de non linéarité ($INL_k$) à partir de la pluralité d'images de contrainte corrigée ($I\varepsilon_k{}^*$) et de la pluralité d'images d'élasticité corrigée ($IE_k{}^*$).

**14.** Procédé selon la revendication 13, dans lequel la valeur de chaque pixel de l'image d'un paramètre de non linéarité (INLk) est déterminée par régression linéaire en contrainte de couples de valeurs, la première valeur de chaque couple correspondant à la valeur d'un même pixel d'une image de contrainte corrigée de la pluralité et la deuxième valeur du couple correspondant à la valeur d'un même pixel d'une image d'élasticité de la pluralité, ladite image de contrainte corrigée de la pluralité et ladite image d'élasticité corrigée de la pluralité étant temporellement successives ou proches, et

dans lequel la régression linéaire en contrainte est établie sur la base de la relation entre la contrainte et l'élasticité suivante :

$$\ln(E) = \ln(E_0) - \frac{A}{\frac{4}{3}\cdot E_0}\cdot\varepsilon$$

où

E est le module d'Young du pixel de l'image d'élasticité corrigée considérée,
$E_0$ est le module d'Young du pixel de la première image d'élasticité corrigée,
ln() est la fonction de logarithme népérien,
$\varepsilon$ est la déformation du pixel de l'image de déformation corrigée considérée, et
A est le paramètre de non linéarité déterminé dudit pixel par ladite régression.

**15. Dispositif d'imagerie (1)** comprenant une sonde ultrasonore (6) et un micro-ordinateur (4) adaptés pour mettre en oeuvre le procédé pour déterminer par ultrasons une image corrigée d'un milieu (2) selon l'une des revendications 1 à 14.


**Patentansprüche**

**1. Verfahren zur Bestimmung eines korrigierten Bildes** eines Mediums mittels Ultraschall, wobei das Verfahren umfasst:

- die Bestimmung einer zeitlichen Abfolge von Bildern des Mediums ($I_k$), wobei k ein Bildindex zwischen 0 und N ist und das erste Bild dieser Abfolge als ohne Deformation angenommen wird,
- die Bestimmung der Bewegung für jedes Bild des Mediums mit dem Index k zwischen 1 und N, wobei die Bewegung zwischen einem Bild des Mediums mit dem Index k ($I_k$) und dem vorhergehenden Bild des Mediums mit dem Index k-1 ($I_{k-1}$) erfolgt,
**dadurch gekennzeichnet, dass** das Verfahren ferner umfasst:

- die Bestimmung einer zeitlichen Abfolge von Bildern mit partieller Deformation ($I\Delta\varepsilon_k$) aus dem Bild des Mediums mit dem Index k ($I_k$) und dem vorhergehenden Bild des Mediums mit dem Index k-1 ($I_{k-1}$),
- die Bestimmung von Bildern mit korrigierter partieller Deformation ($I\Delta\varepsilon_k{}^*$) durch Kompensation der Bilder mit partieller Deformation ($I\Delta\varepsilon_k$) aus den vorhergehenden Bewegungen, d.h. den Bewegungen, die mit den Bildern des Mediums mit Indizes zwischen 1 und k einschließlich verbunden sind, und
- die Bestimmung eines Bildes mit korrigierter Deformation ($I\varepsilon_k$*) durch eine Summe der Bilder mit korrigierter partieller Deformation ($I\Delta\varepsilon_k$*) der Indizes zwischen 1 und k einschließlich, und

wobei die Bestimmung der Bewegung bei dem Index k durchführt wird durch:

- Bestimmung eines Verschiebungsfeldes ($u_k$) zwischen einem Bild des Mediums ($I_k$) und dem vorhergehenden Bild des Mediums ($I_{k-1}$),
- Bestimmung einer geometrischen Bildtransformation ($T_k$) aus dem Verschiebungsfeld ($u_k$), wobei die geometrische Transformation das Verschiebungsfeld ($u_k$) mit einer Anzahl von Parametern von weniger als zehn repräsentiert.

2. Verfahren nach Anspruch 1, wobei das Verschiebungsfeld durch eine Kreuzkorrelation zwischen einem Bild des Mediums ($I_k$) und dem vorhergehenden Bild des Mediums ($I_{k-1}$) oder durch einen Subframe-Tracking-Algorithmus zwischen einem Bild des Mediums ($I_k$) und dem vorhergehenden Bild des Mediums ($I_{k-1}$) berechnet wird.

3. Verfahren nach Anspruch 2, wobei der Tracking-Algorithmus ein Lucas-Kanade-Algorithmus ist.

4. Verfahren nach einem der Ansprüche 1 bis 3, wobei die geometrische Transformation ($T_k$) wenigstens eine Translation oder eine Translation und eine Homothetie oder eine Translation, eine Homothetie und eine Rotation umfasst.

5. Verfahren nach einem der Ansprüche 1 bis 3, wobei die geometrische Transformation ($T_k$) eine Translation, eine Homothetie und eine Rotation umfasst, und wobei die geometrische Transformation in eine Matrixform gebracht wird, wie beispielsweise:

$$T_k = \begin{bmatrix} Hx.cos\theta & -Hz.sin\theta & Tx \\ Hx.sin\theta & Hz.cos\theta & Tz \\ 0 & 0 & 1 \end{bmatrix}$$

mit den folgenden Parametern der geometrischen Transformation ($T_k$) in der Bildebene:

Tx, Ty Translationskoeffizienten,
Hx, Hz Homothetiekoeffizient und
$\Theta$ Drehwinkel mit einer Achse senkrecht zur Bildebene.

6. Verfahren nach Anspruch 1, wobei Parameter der geometrischen Transformation ($T_k$) durch Mittelwerte einer Population von Parametern erhalten werden, die aus Gruppen von Punkten des Verschiebungsfeldes ($u_k$) berechnet werden.

7. Verfahren nach Anspruch 6, wobei:

- eine Gruppe zwischen drei und zehn Punkte des Verschiebungsfeldes ($u_k$) umfasst, und
- die Population mehr als 100 Gruppen beträgt.

8. Verfahren nach Anspruch 1, ferner umfassend:

(e1) die Bestimmung eines Elastizitätsbildes ($IE_k$) des Mediums, und
(e2) die Bestimmung eines Bildes mit korrigierter Elastizität ($IE_k$*) durch Kompensieren des Elastizitätsbildes ($IE_k$) aus vorhergehenden Bewegungen, d.h. Bewegungen, die mit Bildern des Mediums mit Indizes zwischen 1 und k assoziiert sind.

9. Verfahren nach Anspruch 8, wobei das in Schritt (e1) erzeugte Elastizitätsbild ($IE_k$) durch die folgenden Unterschritte erzeugt wird:

(e1.1) einem Anregungsschritt, in dessen Verlauf eine Scherwelle im Medium erzeugt wird, indem wenigstens eine fokussierte Ultraschallwelle ausgesendet wird,
(e1.2) einem Beobachtungsschritt, in dessen Verlauf die Ausbreitung der Scherwelle beobachtet wird, indem eine zeitliche Abfolge von Zwischenbildern des Mediums ($II_{j,k}$) bestimmt wird, wobei j ein Zwischenbildindex zwischen 0 und M ist,
(e1.3) einem Verarbeitungsschritt, in dessen Verlauf das Elastizitätsbild ($IE_k$) aus den Zwischenbildern des Mediums ($II_{i,k}$) und einem Modell der Scherwellenausbreitung bestimmt wird.

**10.** Verfahren nach Anspruch 8, wobei ferner folgendes durchgeführt wird:

- die Bestimmung einer Mehrzahl von Bildern mit korrigierter Deformation ($I\varepsilon_k^*$),
- die Bestimmung einer Mehrzahl von Bildern mit korrigierter Elastizität ($IE_k^*$), wobei die Bilder mit korrigierter Deformation und die Bilder mit Elastizität zeitlich verschachtelt sind, und
- Bestimmung eines Bildes eines Nichtlinearitätsparameters ($INL_k$) aus der Mehrzahl von Bildern mit korrigierter Deformation ($I\varepsilon_k^*$) und der Mehrzahl von Bildern mit korrigierter Elastizität ($IE_k^*$).

**11.** Verfahren nach Anspruch 10, wobei der Wert jedes Pixels des Bildes eines Nichtlinearitätsparameters ($INL_k$) durch lineare Regression in Deformation von Wertepaaren bestimmt wird, wobei der erste Wert jedes Paares dem Wert eines gleichen Pixels eines Bildes mit korrigierter Deformation der Mehrzahl entspricht und der zweite Wert des Paares dem Wert eines gleichen Pixels eines Elastizitätsbildes der Mehrzahl entspricht, wobei das Bild mit korrigierter Deformation der Mehrzahl und das Bild mit korrigierter Elastizität der Mehrzahl zeitlich aufeinander folgen oder nahe beieinander liegen, und wobei die lineare Deformationsregression auf der Grundlage der folgenden Beziehung zwischen Deformation und Elastizität aufgestellt wird:

$$\ln(E) = \ln(E_0) - \frac{A}{\frac{4}{3} \cdot E_0} \cdot \varepsilon$$

wobei gilt:

E ist das Young-Modul des Pixels des betrachteten Bildes mit korrigierter Elastizität,
$E_0$ ist das Elastizitätsmodul des Pixels des ersten Bildes mit korrigierter Elastizität,
ln() ist die neperianische Logarithmusfunktion,
$\varepsilon$ ist die Deformation des Pixels des betrachteten Bildes mit korrigierter Deformation, und
A ist der ermittelte Nichtlinearitätsparameter des Pixels durch die lineare Regression in Deformation.

**12.** Verfahren nach Anspruch 8, wobei ferner folgendes durchgeführt wird:

- die Bestimmung einer zeitlichen Abfolge von Bildern mit korrigierter partieller Belastung ($I\Delta\sigma_k^*$) durch eine Multiplikation des Bildes mit korrigierter partieller Deformation ($I\Delta\varepsilon_k^*$) mit dem Index k und des Bildern mit korrigierter Elastizität ($IE_k^*$) mit dem Index k,
- Bestimmung eines Bildes mit korrigierter Belastung ($I\sigma_k^*$) durch eine Summe der Bilder mit korrigierter partieller Deformation ($I\Delta\sigma_k^*$) der Indizes zwischen 1 und k einschließlich.

**13.** Verfahren nach Anspruch 12, wobei ferner folgendes durchgeführt wird:

- die Bestimmung einer Mehrzahl von Bildern mit korrigierter Belastung ($I\sigma_k^*$),
- die Bestimmung einer Mehrzahl von Bildern mit korrigierter Elastizität ($IE_k^*$), wobei die Bilder mit korrigierter Deformation und die Bilder mit Elastizität zeitlich verschachtelt sind, und
- die Bestimmung eines Bildes eines Nichtlinearitätsparameters ($INL_k$) aus der Mehrzahl von Bildern mit korrigierter Deformation ($I\varepsilon_k^*$) und der Mehrzahl von Bildern mit korrigierter Elastizität ($IE_k^*$).

**14.** Verfahren nach Anspruch 13, wobei der Wert jedes Pixels des Bildes eines Nichtlinearitatsparameters ($INL_k$) durch lineare Regression unter Belastung von Wertepaaren bestimmt wird, wobei der erste Wert jedes Paares dem Wert desselben Pixels eines Bildes mit korrigierter Belastung der Mehrzahl entspricht und der zweite Wert des Paares dem Wert desselben Pixels eines Bildes mit Elastizität der Mehrzahl entspricht, wobei das Bild mit korrigierter Belastung der Mehrzahl und das Bild mit korrigierter Elastizität der Mehrzahl zeitlich aufeinander folgen oder nahe beieinander liegen, und wobei die lineare Regression unter Belastung auf der Grundlage der folgenden Beziehung zwischen Belastung und Elastizität erstellt wird:

$$\ln(E) = \ln(E_0) - \frac{A}{\frac{4}{3} \cdot E_0} \cdot \varepsilon$$

wobei gilt:

E ist das Young-Modul des Pixels des betrachteten Bildes mit korrigierter Elastizität,
$E_0$ ist das Young-Modul des Pixels des ersten Bildes mit korrigierter Elastizität,
ln() ist die neperianische Logarithmusfunktion,
$\varepsilon$ ist die Deformation des Pixels des betrachteten Bildes mit korrigierter Deformation, und
A ist der ermittelte Nichtlinearitätsparameter des Pixels durch die Regression.

15. **Bildgebungsvorrichtung** (1), umfassend eine Ultraschallsonde (6) und einen Mikrocomputer (4), die zur Durchführung des Verfahrens zur Bestimmung eines korrigierten Bildes eines Mediums (2) mittels Ultraschall nach einem der Ansprüche 1 bis 14 ausgebildet sind.

## Claims

1. **Method for ultrasound determination of a corrected image** of a medium, the method comprising:

   - determination of a temporal succession of images of the medium ($I_k$), k being an image index between 0 and N, and the first image of said succession being assumed to be without strain,
   - determination of the movement for each image of the medium of index k between 1 and N, the movement being between an image of the medium of index k ($I_k$) and the preceding image of the medium of index k-1 ($I_{k-1}$),
   - determination of a temporal succession of partial strain images ($I\Delta\varepsilon_k$), based on the image of the medium of index k ($I_k$) and the preceding image of the medium of index k-1 ($I_{k-1}$),
   - determination of corrected partial strain images ($I\Delta\varepsilon_k{}^*$), by compensation of said partial strain images ($I\Delta\varepsilon_k$) based on preceding movements, meaning the movements associated with the images of the medium having indices between 1 and k inclusive, and
   - determination of a corrected strain image ($I\varepsilon_k{}^*$) by summation of the corrected partial strain images ($I\Delta\varepsilon_k{}^*$) of indices between 1 and k inclusive, and

   wherein the determination of the movement of index k is carried out by:

   - determination of a field of displacement ($u_k$) between an image of the medium ($I_k$) and the preceding image of the medium ($I_{k-1}$),
   - determination of an image geometric transformation ($T_k$) based on said field of displacement ($u_k$), said geometric transformation representing the field of displacement ($u_k$) with fewer than ten parameters.

2. Method according to claim 1, wherein the field of displacement is calculated by intercorrelation between an image of the medium (Ik) and the preceding image of the medium ($I_{k-1}$), or by an algorithm for tracking sub-images between an image of the medium (Ik) and the preceding image of the medium (Ik-1).

3. Method according to claim 2, wherein the tracking algorithm is a Lucas-Kanade algorithm.

4. Method according to one of claims 1 to 3, wherein the geometric transformation (Tk) comprises at least one translation, or a translation and a homothety, or a translation, a homothety, and a rotation.

5. Method according to one of claims 1 to 3, wherein the geometric transformation (Tk) comprises a translation, a homothety, and a rotation, and wherein the geometric transformation is put in matrix form such that:

$$T_k = \begin{bmatrix} Hx.\cos\theta & -Hz.\sin\theta & Tx \\ Hx.\sin\theta & Hz.\cos\theta & Tz \\ 0 & 0 & 1 \end{bmatrix}$$

with the following parameters of the geometric transformation (Tk) in the image plane:

Tx, Ty translation coefficients,
Hx, Hz coefficient of homothety, and
$\theta$ angle of rotation of axis perpendicular to the image plane.

6. Method according to claim 1, wherein the parameters of the geometric transformation (Tk) are obtained by median values of a population of parameters that are calculated based on groups of points in the field of displacement (uk).

7. Method according to claim 6, wherein:

- a group comprises between three and ten points of the field of displacement ($u_k$), and
- the population is greater than one hundred groups.

8. Method according to claim 1, further comprising:

(e1) determination of an elasticity image ($IE_k$) of the medium, and
(e2) determination of a corrected elasticity image ($IE_k^*$), by compensation of said elasticity image ($IE_k$) based on the preceding movements, meaning the movements associated with the images of the medium having indices between 1 and k inclusive.

9. Method according to claim 8, wherein the elasticity image (IEk) produced in step (e1) is generated by the following sub-steps:

(e1.1) an excitation step during which a shear wave is generated in the medium, by causing at least one focused ultrasonic wave to be emitted,
(e1.2) an observation step during which the propagation of the shear wave is observed by determining a temporal succession of intermediate images of the medium ($II_{j,k}$), j being an intermediate image index between 0 and M,
(e1.3) a processing step during which the elasticity image ($IE_k$) is determined based on said intermediate images of the medium ($II_{j,k}$) and based on a shear wave propagation model.

10. Method according to claim 8, wherein the following are further carried out:

- determination of a plurality of corrected strain images ($I\varepsilon_k^*$),
- determination of a plurality of corrected elasticity images ($IE_k^*$), said corrected strain images and said elasticity images being temporally interlaced, and
- determination of an image of a nonlinearity parameter ($INL_k$), based on the plurality of corrected strain images ($I\varepsilon_k^*$) and the plurality of corrected elasticity images ($IE_k^*$) .

11. Method according to claim 10, wherein the value of each pixel of the image of a nonlinearity parameter ($INL_k$) is determined by strain linear regression of pairs of values, the first value of each pair corresponding to the value of a same pixel of a corrected strain image among the plurality and the second value of the pair corresponding to the value of a same pixel of an elasticity image among the plurality, said corrected strain image among the plurality and said corrected elasticity image among the plurality being temporally successive or temporally close, and
wherein the strain linear regression is established on the basis of the following relation between strain and elasticity:

$$\ln(E) = \ln(E_0) - \frac{A}{\frac{4}{3} \cdot E_0} . \varepsilon$$

where

E is the Young's modulus of the pixel of the corrected elasticity image considered,
$E_0$ is the Young's modulus of the pixel of the first corrected elasticity image,
ln() is the natural logarithm function,
$\varepsilon$ is the strain of the pixel of the corrected strain image considered, and

A is the nonlinearity parameter of said pixel, determined by said strain linear regression.

12. Method according to claim 8, wherein the following are further carried out:

- determination of a temporal succession of corrected partial stress images ($I\Delta\sigma_k^*$) by multiplying the corrected partial strain image ($I\Delta\varepsilon_k^*$) of index k and the corrected elasticity image ($IE_k^*$) of index k,
- determination of a corrected stress image ($I\sigma_k^*$) by summation of the corrected partial strain images ($I\Delta\sigma_k^*$) having indices between 1 and k inclusive.

13. Method according to claim 12, wherein the following are further carried out:

- determination of a plurality of corrected stress images ($I\sigma_k^*$),
- determination of a plurality of corrected elasticity images ($IE_k^*$), said corrected strain images and said elasticity images being temporally interlaced, and
- determination of an image of a nonlinearity parameter ($INL_k$), based on the plurality of corrected stress images ($I\varepsilon_k^*$) and the plurality of corrected elasticity images ($IE_k^*$).

14. Method according to claim 13, wherein the value of each pixel of the image of a nonlinearity parameter (INLk) is determined by stress linear regression of pairs of values, the first value of each pair corresponding to the value of a same pixel of a corrected stress image among the plurality and the second value of the pair corresponding to the value of a same pixel of an elasticity image among the plurality, said corrected stress image among the plurality and said corrected elasticity image among the plurality being temporally successive or temporally close, and wherein the stress linear regression is established on the basis of the following relation between stress and elasticity:

$$\ln(E) = \ln(E_0) - \frac{A}{\frac{4}{3} \cdot E_0} \cdot \varepsilon$$

where

E is the Young's modulus of the pixel of the corrected elasticity image considered,
$E_0$ is the Young's modulus of the pixel of the first corrected elasticity image,
ln() is the natural logarithm function,
$\varepsilon$ is the strain of the pixel of the corrected strain image considered, and
A is the nonlinearity parameter determined for said pixel by said regression.

15. **Imaging device (1)** comprising an ultrasound probe (6) and a microcomputer (4) which are suitable for implementing the method for ultrasound determination of a corrected image of a medium (2) according to one of claims 1 to 14.

# FIG. 1

# FIG. 2A

# FIG. 2B

# FIG. 2C

# FIG. 3

| | |
|---|---|
| Détermination d'une succession temporelle d'images du milieu ($I_k$) | d1 |
| Détermination du mouvement entre les images du milieu | d2 |
| Détermination des images de déformation partielle ($I\Delta\varepsilon_k$) | d3 |
| Détermination des images de déformation partielle corrigée ($I\Delta\varepsilon_k^*$) à partir des mouvements précédents | d4 |
| Calcul d'une image de déformation corrigée ($I\varepsilon_k^*$) par cumul des images de déformation partielle corrigée ($I\Delta\varepsilon_k^*$) | d5 |

# FIG. 4

Détermination d'une image
d'élasticité (IE$_k$) —e1

↓

Détermination d'une image
d'élasticité corrigée (IE$_k^*$)
à partir des mouvements précédents —e2

# FIG. 5

NL$_a$

Détermination de plusieurs images
de déformation corrigée (Iε$_k^*$) —NL1a

↓

Détermination de plusieurs images
d'élasticité corrigée (IE$_k^*$) —NL2a

↓

Détermination d'une image corrigée
d'un paramètre de non linéarité —NL3a

# FIG. 6

$$\ell n(E) = \ell n(E_0) - \frac{A}{\frac{4}{3}E_0} \, \varepsilon$$

# FIG. 7A

| | |
|---|---|
| Détermination d'une succession temporelle d'images de contrainte partielle corrigée $(I\Delta\sigma_k^*)$ $(I\Delta\sigma_k^*) = (I\Delta\varepsilon_k^*) \cdot (IE_k^*)$ | C1 |

$\downarrow$

| | |
|---|---|
| Détermination d'une image de contrainte corrigée $(I\sigma_k^*)$ par cumul des images de contrainte partielle corrigée $(I\Delta\sigma_k^*)$ | C2 |

# FIG. 7B

$NL_b$

| | |
|---|---|
| Détermination de plusieurs images de contrainte corrigée $(I\sigma_k^*)$ | NL1b |

$\downarrow$

| | |
|---|---|
| Détermination de plusieurs images d'élasticité corrigée $(IE_k^*)$ | NL2b |

$\downarrow$

| | |
|---|---|
| Détermination d'une image corrigée d'un paramètre de non linéarité | NL3b |

# FIG. 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2005252295 A
- US 2015146953 A
- US 2009234230 A

**Littérature non-brevet citée dans la description**

- **O'DONNELL et al.** Internal displacement and strain imaging using speckle tracking. *IEEE transactions on ultrasonic, ferroelectrics, and frequency control,* Mai 1994, vol. 41 (3), 314-325
- **OPHIR et al.** Elastography: a quantitative method for imaging the elasticity of biological tissues. *Ultrasonic imaging.,* 1991, vol. 13, 111-134
- **J-Y BOUGUET.** Pyramidal Implementation of the Lucas Panade Feature Tracter. Description of the algorithm. Intel Corp,
- **GENNISSON et al.** Acoustoelasticity in soft solids : Assesment of the non-linear shear modulus with the acoustic radiation force. *J. Acoust. Soc. Am,* Décembre 2007, vol. 122, 3211-3219
- **H. LATORRE-OSSA et al.** Quantitative Imaging of Nonlinear Shear Modulus by Combining Static Elastography and Shear Wave Elastography. *IEEE Transactions on Ultrasonics, Ferroelectrics, and Frequency Control,* vol. 51 (4), 833-839